(19) Europäisches Patentamt
European Patent Office
Office européen des brevets

(11) **EP 1 317 668 B1**

(12) **EUROPÄISCHE PATENTSCHRIFT**

(45) Veröffentlichungstag und Bekanntmachung des Hinweises auf die Patenterteilung:
**06.10.2004   Patentblatt 2004/41**

(51) Int Cl.[7]: **G01N 31/10**, B01J 23/755, B01D 53/86

(21) Anmeldenummer: **01978361.2**

(22) Anmeldetag: **12.09.2001**

(86) Internationale Anmeldenummer:
**PCT/EP2001/010535**

(87) Internationale Veröffentlichungsnummer:
**WO 2002/023182 (21.03.2002 Gazette 2002/12)**

(54) **VERFAHREN ZUR PYROLYSE VON QUECKSILBER(II)CHLORID ZUR ANSCHLIESSENDEN BESTIMMUNG DES QUECKSILBERS**

METHOD FOR THE PYROLYSIS OF MERCURIC CHLORIDE FOR THE SUBSEQUENT ANALYSIS OF THE MERCURY

PROCEDE POUR LA PYROLYSE DE CHLORURE MERCURIQUE POUR LE DOSAGE CONSECUTIF DU MERCURE

(84) Benannte Vertragsstaaten:
**AT BE CH CY DE DK ES FI FR GB GR IE IT LI LU MC NL PT SE TR**

(30) Priorität: **13.09.2000   DE 10045212**

(43) Veröffentlichungstag der Anmeldung:
**11.06.2003   Patentblatt 2003/24**

(73) Patentinhaber:
• **Seefelder Messtechnik Gmbh & Co, Vertriebs KG**
  **82229 Seefeld (DE)**
• **Forschungszentrum Karlsruhe GmbH**
  **76344 Eggenstein-Leopoldshafen (DE)**

(72) Erfinder:
• **WINKLER, Wilfried, M.**
  **82041 Oberbiberg (DE)**

• **BERKHAHN, Wolfgang**
  **88662 Überlingen (DE)**
• **SCHRADER, Christoph**
  **76344 Eggenstein-Leopoldshafen (DE)**
• **PAUR, Hanns, Rudolf**
  **76139 Karlsruhe (DE)**

(74) Vertreter: **Müller, Bernhard, Dr.**
  **H. Schmidt & B. Müller Patentanwälte**
  **Graf-Toerring-Strasse 45**
  **82229 Seefeld (DE)**

(56) Entgegenhaltungen:
  **DE-A- 3 931 891          DE-A- 4 143 170**
  **US-A- 4 758 519**

**Beschreibung**

Die Erfindung betrifft ein Verfahren zur Bestimmung von Quecksilber. Insbesondere betrifft die Erfindung die Bestimmung von Quecksilber in quecksilberhaltigen Rauchgasen, z. B. in Abgasen von Müllverbrennungsanlagen.

Die Abgase von Müllverbrennungsanlagen enthalten häufig Quecksilber in Form von $HgCl_2$ und Hg (0), wobei diese Abgase in der Regel auch erhebliche HCl-Konzentrationen enthalten.

Da für die Emission von Quecksilber strenge Grenzwerte vorgeschrieben sind, ist die laufende Überwachung der Abgase erforderlich. Hierzu stehen Quecksilberanalysatoren zur Verfügung, in denen elementares Quecksilber [Hg(0)] spektrophotometrisch gemessen wird. Hierzu ist es erforderlich, das in den Abgasen enthaltene $HgCl_2$ in Hg(0) überzuführen.

US-A-4758519 offenbart eine Methode für die kontinuierliche Analyse des Quecksilbergehalts eines Gases, das Quecksilberverbindungen enthält. Das Gas wird mit einem Reduktionsmittel (ausgewählt aus einem Metall der Gruppen I, II oder IV) in Kontakt gebracht um das Quecksilber in seine elementare Form zu führen bevor es dem Analysator zugeführt wird. Es wird darauf hingewiesen, dass die Anwesenheit von HCl und/oder SO2 im Gas das Verfahren nicht behindern.

Im Rahmen von Vorarbeiten, die zur vorliegenden Erfindung führten, wurde jedoch festgestellt, dass für eine Pyrolyse von $HgCl_2$ zu Hg(0) gemäß der nachstehenden Reaktionsgleichung (1) eine Temperatur von etwa 900 °C notwendig ist. Es tritt jedoch bereits ab 800 °C eine Rückreaktion von Hg(0) mit HCl und $O_2$ zu $HgCl_2$ und $H_2O$ auf (Reaktionsgleichung (2)).

$$(1) \qquad HgCl_2 \Leftrightarrow Hg(0) + Cl_2$$

$$(2) \qquad Hg(0) + 2\ HCl + 1/2\ O_2 \Leftrightarrow HgCl_2 + H_2O$$

Aus der Reaktionsgleichung (2) ist ersichtlich, dass die Pyrolysetemperatur t höchstens 800 °C betragen sollte.

Aufgabe der Erfindung ist es, ein Verfahren zur Bestimmung von Quecksilber bereitzustellen, bei dem die vorgeschaltete Pyrolyse bei Temperaturen von ≤800 °C abläuft.

Erfindungsgemäß wurde nunmehr festgestellt, dass sich elementares Nickel als Katalysatormaterial für die Pyrolyse von $HgCl_2$ zu Hg(0) eignet. Als besonders geeignet haben sich dabei Nickellegierungen mit einem Nickelgehalt von etwa 50 bis 90 %, vorzugsweise 60 bis 85 % und insbesondere 70 bis 80 % erwiesen. Ein bevorzugtes Beispiel für eine derartige Legierung ist Inkonel 600 mit der deutschen Werkstoffnummer 2.4816. Dieser Stahl ist säure- und hochtemperaturbeständig. Er enthält 15 % Cr, 75 % Ni und 8 % Fe.

Der Katalysator wird vorzugsweise in Form von gesintertem Material, das gleichzeitig eine Rauchgasfilterung bewirkt, bereitgestellt. Möglich ist auch die Verwendung von Füllkörpern, die mit einem derartigen Nickelmaterial überzogen sind. Auch die Verwendung von feinen Drahtsieben kommt in Betracht.

Nachstehend wird die Erfindung anhand von Diagrammen näher erläutert.

Es zeigen:

Fig. 1 ein Diagramm zur Erläuterung der Pyrolyse von $HgCl_2$ in Abhängigkeit von der Temperatur;
Fig. 2 den Einfluss von Feuchtigkeit auf die Pyrolyse von $HgCl_2$;
Fig. 3 den Einfluss der HCl-Konzentration auf die Pyrolyse von $HgCl_2$ und
Fig. 4 den Einfluss von $SO_2$ auf die Pyrolyse von $HgCl_2$.
Fig. 5 das Ergebnis von Pyrolyseversuchen in Gegenwart und Abwesenheit von Ni-Katalysator.

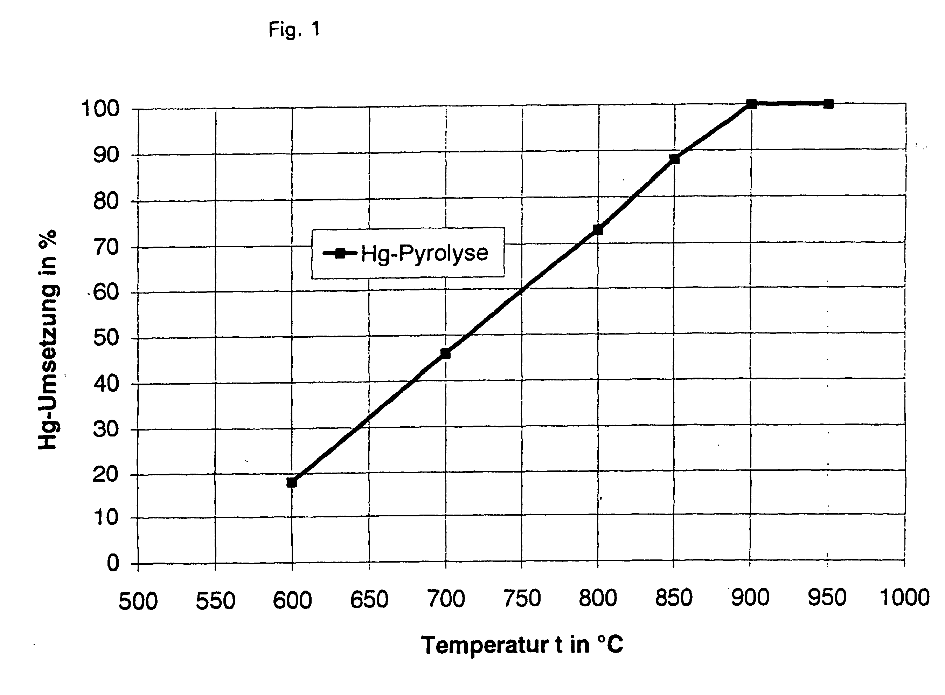

Fig. 1 zeigt, bei welcher Temperatur die vollständige Pyrolyse von $HgCl_2$ zu Hg(0) abläuft. Dabei wurde als Trägergas für sämtliche Versuche Stickstoff mit 8 Vol-% $O_2$ und 15 Vol-% $H_2O$ verwendet. Der Volumenstrom betrug 150 Liter/h und die Quecksilberkonzentration wurde auf ≈ 100 µg/m³ eingestellt. Wie aus Fig. 1 ersichtlich ist, beginnt die Pyrolyse bei etwa 600 °C und ist bei einer Ofentemperatur von 900 °C vollständig.

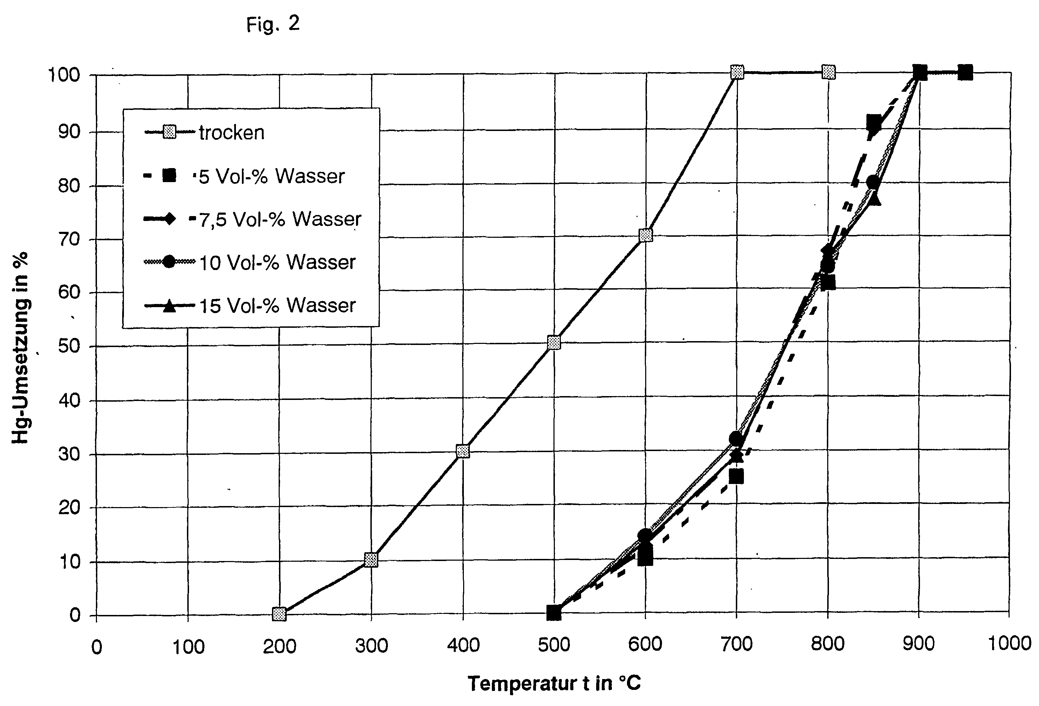

Fig. 2 zeigt den Einfluss von Wasserdampf auf die Pyrolyse von $HgCl_2$. Für vier verschiedene Wasserdampfkonzentrationen zwischen 5 und 15 Vol-% sowie in Abwesenheit von Wasserdampf wurde die Abhängigkeit der Pyrolyse von der Temperatur ermittelt. Es ist ersichtlich, dass Feuchtigkeit generell einen.sehr großen Einfluss auf die Pyrolyse hat. Die Temperatur, die man zur vollständigen Pyrolyse benötigt, wird durch die Anwesenheit von Wasserdampf von etwa 700 auf 900 °C verschoben. Im untersuchten Bereich mit einem Wasserdampfgehalt von 5 bis 15 Vol-% konnte kein Einfluss des Feuchtigkeitsgehalts auf die Pyrolyse festgestellt werden.

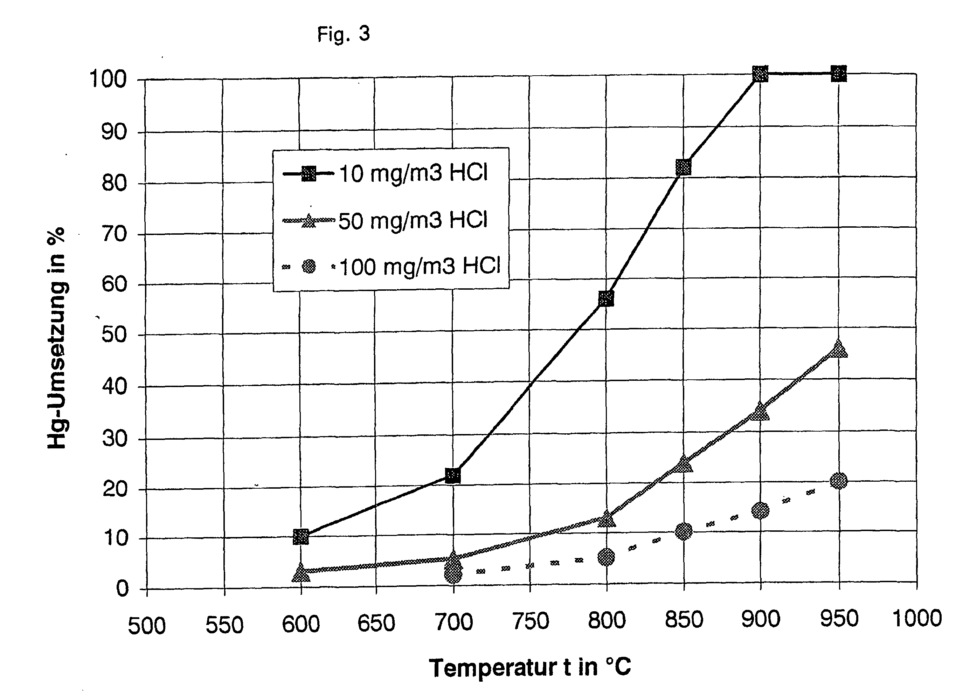

Fig. 3 erläutert den Einfluss von unterschiedlichen HCl-Konzentrationen zwischen 10 und 100 mg/m³ auf die Pyrolyse. Dabei wurde dem Trägergas $HgCl_2$ zudosiert, um reale Bedingungen, wie sie beispielsweise in Müllverbrennungsanlagen herrschen, zu simulieren. Bei HCl-Konzentrationen von 50 und 100 mg/m³ konnte keine vollständige Pyrolyse erreicht werden, dagegen ist bei einer HCl-Konzentration von 10 mg/m³ bei einer Temperatur von 900 °C noch eine vollständige Pyrolyse möglich.

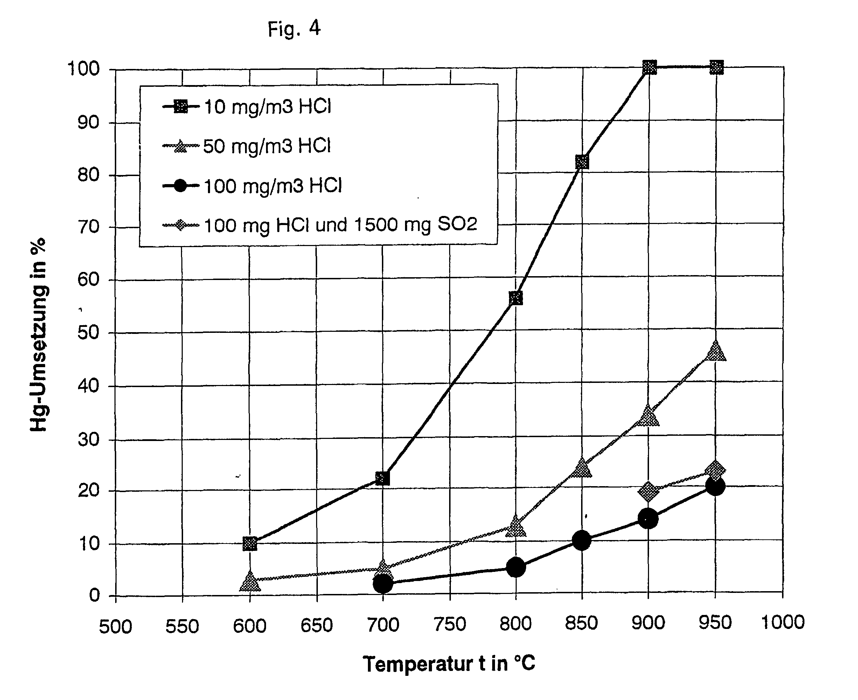

Fig. 4 zeigt den Einfluss von $SO_2$ auf die Pyrolyse von $HgCl_2$. Dabei wurden dem Trägergas (mit einer $Hg^{2+}$-Konzentration von 100 µg/m³) 100 mg/m³ HCl und 1500 mg/m³ $SO_2$ zugemischt. Wie aus Fig. 4 ersichtlich ist, erreicht man bei einer Ofentemperatur von 950 °C mit und ohne $SO_2$ etwa die selbe Hg(0)-Konzentration nach der Pyrolyse. Daraus lässt sich der Schluss ableiten, dass $SO_2$, das unter Praxisbedingungen in derartigen Rauchgasen enthalten ist, keinen Einfluss auf die Pyrolyse hat.

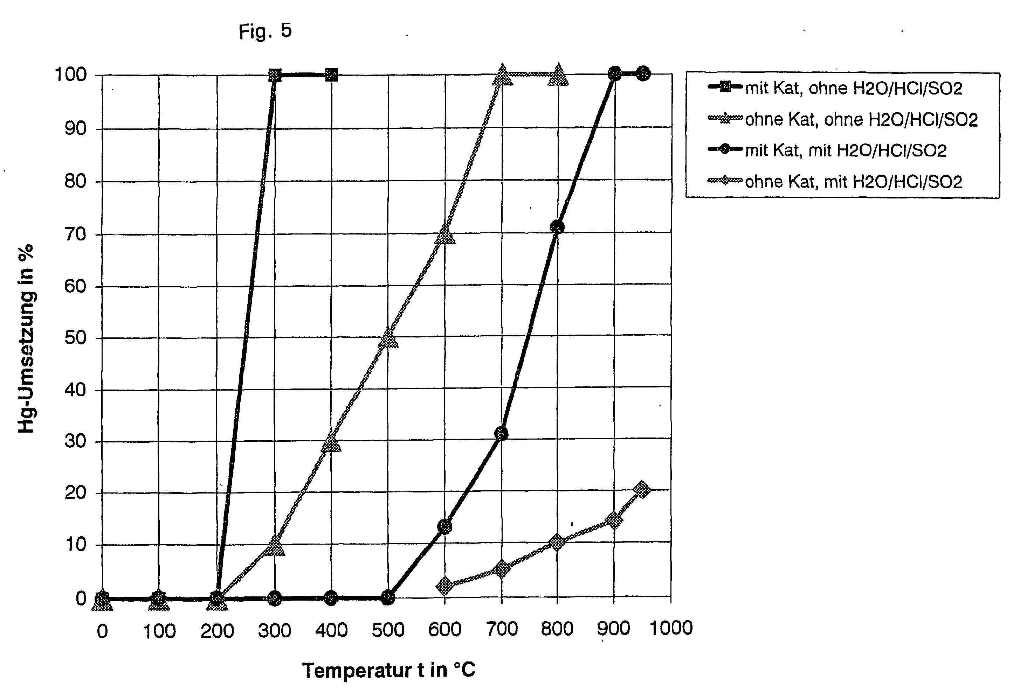

Fig. 5 zeigt das Ergebnis von Pyrolyseversuchen mit und ohne Ni-Katalysator. Dabei dienten vernikkelte Füllkörper als Katalysatoren. Bei einer $Hg^{2+}$-Konzentration von 100 µg/m$^3$ wurden Versuche ohne $H_2O$, HCl und $SO_2$ und mit 8 Vol-% $H_2O$, 100 mg/m$^3$ HCl und 1500 mg/m$^3$ $SO_2$ durchgeführt. Ohne $H_2O$, HCl und $SO_2$ konnte in Anwesenheit von Katalysator die Temperatur für die vollständige Pyrolyse von 700 auf 300 °C gesenkt werden. Mit $H_2O$, 100 mg/m$^3$ HCl und 1500 mg/m$^3$ $SO_2$ konnte in Anwesenheit des Katalysators bei diesen Bedingungen bei 900 °C eine vollständige Pyrolyse erreicht werden, während ohne Katalysator die Hg-Umsetzung nur 15 % betrug. Bei HCl-Konzentrationen unter 100 mg/m$^3$ liegt die Temperatur für die vollständige Pyrolyse entsprechend niedriger, so dass die angestrebte Temperatur von 800 °C erreicht werden kann. Es ergibt sich, dass durch Verwendung des Katalysators bei niedrigeren Temperaturen eine höhere Hg-Umsetzung erreicht werden kann.

**Patentansprüche**

1. Verfahren zur Pyrolyse von Quecksilber (II) chlorid in Gasen mit einem Gehalt an $HgCl_2$, Hg(0) und HCl, **dadurch gekennzeichnet, dass** man die Pyrolyse von $HgCl_2$ in Gegenwart von Nickel als Katalysator durchführt.

2. Verfahren nach Anspruch 1, **dadurch gekennzeichnet, dass** man als Nickelkatalysator eine Nikkellegierung mit einem Nickelgehalt von 50 bis 90 % verwendet.

3. Verfahren nach Anspruch 1, **dadurch gekennzeichnet, dass** man als Nickelkatalysator Inkonel (Werkstoffnummer 2.4816) verwendet.

4. Verwendung von Nickel als Katalysator bei der Pyrolyse von Quecksilberverbindungen in Gegenwart von HCl.

5. Verwendung nach Anspruch 4, **dadurch gekennzeichnet, dass** das Nickelmaterial als gesintertes Material vorliegt.

**Claims**

1. Method for the pyrolysis of mercuric chloride in gases containing $HgCl_2$, Hg(0) and HCl, **characterized in that** one carries out the pyrolysis of $HgCl_2$ in the presence of nickel as a catalyst.

2. Method according to claim 1, **characterized in that** one utilizes as a nickel catalyst a nickel alloy having a nickel content of 50 to 90 %.

3. Method according to claim 1, **characterized in that** one uses as a nickel catalyst Inkonel (Industrial Material Code 2.4816).

4. Use of nickel as a catalyst during the pyrolysis of mercury compounds in the presence of HCl.

5. Use according to claim 4, **characterized in that** the nickel material is present as sintered material.

**Revendications**

1. Procédé pour la pyrolyse du chlorure de mercure (II) dans des gaz ayant une teneur en $HgCl_2$, Hg(0) et HCl **caractérisé en ce que** l'on effectue la pyrolyse du $HgCl_2$ en présence de nickel comme catalyseur.

2. Procédé selon la revendication 1, **caracterisé en ce que** comme catalyseur nickel, on utilise un alliage de nickel ayant une teneur en nickel de 50 à 90 %.

3. Procédé selon la revendication 1, **caractérisé en ce que** comme catalyseur de nickel on utilise de l'inconel (Numéro de matériau 2.4816).

4. Utilisation de nickel comme catalyseur lors de la pyrolyse de composés de mercure en présence de HCl.

5. Utilisation selon la revendication 4, **caractérisée en ce que** le matériau nickel se présente sous la forme de matériau fritté.

Fig. 1

EP 1 317 668 B1

Fig. 2

Fig. 3

Legend:
- 10 mg/m3 HCl
- 50 mg/m3 HCl
- 100 mg/m3 HCl

X-axis: **Temperatur t in °C**

Y-axis: **Hg-Umsetzung in %**

EP 1 317 668 B1

Fig. 4

EP 1 317 668 B1

Fig. 5

mit Kat, ohne H2O/HCl/SO2
ohne Kat, ohne H2O/HCl/SO2
mit Kat, mit H2O/HCl/SO2
ohne Kat, mit H2O/HCl/SO2

Hg-Umsetzung in %

Temperatur t in °C